# EUROPEAN PATENT APPLICATION

(11) **EP 4 442 205 A1**
(43) Date of publication of application: **09.10.2024**
(21) Application number: 23168235.2
(22) Date of filing: 17.04.2023
(51) Int. Cl.: A61B 8/00, A61B 8/08

(54) **ASSESSING ACQUISITION QUALITY OF A 3D ULTRASOUND SCAN**

(30) Priority: 06.04.2023 US 202363457423 P
(71) Applicant: Koninklijke Philips N.V., 5656 AG Eindhoven (NL)
(72) Inventor: ROBERT, Jean-luc francois-marie, Eindhoven (NL); ROESSL, Ewald, Eindhoven (NL)
(74) Representative: Philips Intellectual Property & Standards

(57) **Abstract**

Proposed concepts aim to provide schemes, solutions, concepts, designs, methods and systems pertaining to assessing acquisition quality of a scan of a subject by a 3D ultrasound scanner. Specifically, assessment of image quality in 3D is achieved based on a coherence value of the image data generalized in an elevation dimension (rather than an azimuth dimension used for 2D ultrasound data). Thus, image quality values are determined for different parts of the 3D region based on the coherence factor of the associated ultrasound image data. From the image quality values, a volume of the 3D region that is adequately scanned may be determined. In this way, feedback regarding the acquisition quality of the 3D ultrasound scan may be provided.

## Description

### FIELD OF THE INVENTION

The present invention relates to the field of assessing scanner acquisition quality, and more particularly to the field of assessing acquisition quality of a scan by a 3D ultrasound scanner.

### BACKGROUND OF THE INVENTION

The quality of an image acquired by a 3D ultrasound scan depends on a number of factors. Proper positioning and orientation of the transducer of the ultrasound scanner is required in order to ensure that a desired region of a subject (i.e. region of interest) is adequately imaged. Further, the desired region may not be imaged when there is a blockage (e.g. a bone between the transducer and the region of interest).

Providing feedback regarding the quality of an acquired scan of a subject enables adjustments to be made to improve said quality. This may be useful for training purposes, ensuring the sonographer is provided with information regarding how to improve the scan. Furthermore, such feedback may be useful in real-time scanning situations in order to minimize the amount of time taken to perform an ultrasound scan, which may be inconvenient and uncomfortable for the subject.

In the case of a 2D ultrasound scan, it may be straightforward to directly visualize the scan, so that feedback can be performed. That is, in 2D, the sonographer can directly consult the image to determine the impact of adjustments on the acoustic window. However, this proves to be more difficult in 3D, particularly as part of the volume may have a good acoustic window, while another part may suffer from a bad acoustic window.

### SUMMARY OF THE INVENTION

The invention is defined by the claims.

According to examples in accordance with an aspect of the invention, there is provided a method for assessing acquisition quality of a scan of a subject by a 3D ultrasound scanner, comprising:
obtaining ultrasound image data associated with a 3D region of a subject;
determining, for each of a plurality of different parts of the 3D region, an image quality value indicating a quality of the ultrasound image data associated with the part of the 3D region, each image quality value based on a coherence factor of the associated ultrasound image data generalized to an elevation dimension; and
determining a volume of the 3D region that is adequately scanned based on identifying parts of the 3D region associated with image quality values meeting a predetermined requirement.

The invention provides a means by which a volume of a 3D region (i.e. a region of interest) that is adequately scanned (i.e. of a minimum required acquisition quality) can be determined. That is, the invention provides a way of determining/identifying a volume of a subject of a 3D ultrasound scan that can be imaged with sufficient quality from the associated ultrasound image data.

While it may be straightforward to simply produce the image from a 2D ultrasound scan (and thus provide a means by which a sonographer can determine adequacy of the imaging data), this is more problematic for a 3D scan, as it is impractical to render and display all slices of a 3D region. Indeed, such a rendering would also be difficult and/or confusing for a user to understand.

Proposed embodiments seek to overcome this problem by determining a volume of a 3D region that is adequately scanned. Specifically, this is achieved by determining and/or calculating quality values for different parts of the 3D region. The quality value is based on a coherence factor - a well-known metric in ultrasound imaging. However, the coherence factor is based on focused transmission (i.e. 2D ultrasound scanning), rather than unfocused beams in azimuth as in 3D ultrasound scanning. Thus, embodiments are based on the realization that a coherence factor may be used to characterize the quality of the ultrasound image data of a part of a 3D volume by generalizing the coherence factor to an elevation dimension.

Put another way, embodiments may be based on the realization that an image quality can be assessed using a coherence factor if the coherence factor is generalized to an elevation dimension. This may enable a straightforward measure of the quality of the ultrasound image data associated with different parts of the 3D region of the subject, which may in turn be used to determine a region that has been adequately scanned.

The volume that is adequately scanned may be presented to the user/sonographer, or an indication of such a volume may be provided. As a result, embodiments may provide the advantage of an improved ultrasound scan (as relevant feedback may be provided), or a time taken to complete the scan may be reduced. Accordingly, time of the sonographer and subject may be saved.

In some embodiments, the method may further comprise generating a signal comprising information for displaying a visual indication describing the volume of the 3D region.

Accordingly, the advantages enabled by the determination/identification of the volume of the 3D region that is adequately scanned are realised. That is, a visual indication may be displayed describing the volume of the 3D region, wither reassuring the user/sonographer that a volume of interest has been adequately scanned, or prompting them to adjust the scan to adequately scan a volume of interest.

Furthermore, said visual indication may comprise a set of lines representing boundaries of the volume.

One simple way in which feedback may be provided is with a set of lines delimiting the volume that is adequately scanned. This may be quickly and easily interpreted by the user/sonographer to gain a clear understanding of the volume that is/has been adequately scanned.

In addition, said visual indication may further comprise an image representing at least a part of the 3D region of the subject overlaid by the set of lines.

By overlaying the above lines on a visual representation of the subject (i.e. a simplified representation of organs/a region on the subject), the ease by which the user/sonographer may understand the volume that is adequately scanned may be increased.

In some embodiments, the predetermined requirement may be based on at least one of a contrast value, a resolution value, a distortion value, a noise value, a sharpness value, and an artifact density value.

By basing the predetermined requirement on one of these factors, the quality of the ultrasound scan data may be improved.

In additional embodiments, identifying parts of the 3D region may comprise assessing each of the image quality values based on the predetermined requirement.

One method by which adequate regions may be assessed is by individually comparing each image quality value against the predetermined requirement(s).

In other embodiments, identifying parts of the 3D region may comprise determining sets of image quality values, each set of image quality values associated with parts of the 3D region in a different image direction; for each image direction. processing the associated set of image quality values to determine a set quality value indicating a quality of the ultrasound image data in the image direction; and determining the volume of the subject that is adequately scanned based on identifying image directions associated with set quality values meeting the predetermined requirement.

In other words, an alternative means by which the regions may be assessed as adequate is by comparing the image quality values in each image direction (i.e. in directions extending from the transducer). This may ensure that a visualization (e.g. by means of a set of lines delimiting the adequately imaged volume) is straightforward to render.

Some embodiments of the method may further comprise processing the ultrasound image data to determine a position of a structure of the subject in the 3D region obstructing ultrasound imaging of the subject, wherein determining the volume of the 3D region is further based on the determined position of the structure.

Another piece of information useful for determining a 3D region that is adequately scanned is the presence of a structure of the subject. A structure of the subject may obscure the scanner, and thus result in a low quality image. Thus, by determining and using the position of structure(s) of the subject, a more meaningful adequately scanned 3D region can be determined.

Specifically, determining the position of the structure may comprise processing B-mode components of the ultrasound image data based on an image analysis algorithm, or processing channel components of the ultrasound image data based on a signal processing algorithm.

In some embodiments, the structure may comprise at least one rib of the subject.

Further embodiments of the method may further comprise processing the ultrasound image data to determine a position of a transducer of a 3D ultrasound scanner used to acquire the ultrasound image data relative to the 3D region of the subject; wherein determining the volume of the 3D region is further based on the determined position of the transducer.

A further piece of information useful for determining a 3D region that is adequately scanned is the positioning and/or orientation of the transducer of the scanner. Thus, by determining and using the position of the transducer, a more meaningful adequately scanned 3D region can be determined.

Additionally, each of the plurality of different parts of the 3D region of the subject may be voxels of the 3D region of the subject.

In particular, obtaining the ultrasound image data may comprise acquiring the ultrasound image data from a 3D ultrasound scanning session in real-time, and wherein determining the volume is performed during the scanning session.

By performing the determination of the adequately scanned volume in real-time, a user/sonographer may adjust the scanning apparatus accordingly to ensure that the region of interest to them is adequately scanned (without having to perform multiple individual scanning sessions, and assessing the quality of the image between each session).

According to examples in accordance with another aspect of the invention, there is provided a computer program comprising computer program code means adapted, when said computer program is run on a computer, to implement the method of any embodiment of the invention.

According to additional examples in accordance with an aspect of the invention, there is provided a system for assessing acquisition quality of a scan of a subject by a 3D ultrasound scanner, comprising:
an interface configured to obtain ultrasound image data associated with a 3D region of a subject; and
a processor configured to:
   determine, for each of a plurality of different parts of the 3D region, an image quality value indicating a quality of the ultrasound image data associated with the part of the 3D region, each image quality value based on a coherence factor of the associated ultrasound image data generalized to an elevation dimension; and
   determine a volume of the 3D region that is adequately scanned based on identifying parts of the 3D region associated with image quality values meeting a predetermined requirement.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a potential overlay to be presented to a user showing an extent of a 3D volume that is adequately scanned;
Fig. 2 presents a flow diagram of a method for assessing acquisition quality of a scan of a subject by a 3D ultrasound scanner according to embodiments of the invention; and
Fig.3 presents a simplified block diagram of a system for assessing acquisition quality of a scan of a subject by a 3D ultrasound scanner according to another embodiment; and
Fig.4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods and systems pertaining to assessing acquisition quality of a scan of a subject by a 3D ultrasound scanner. Specifically, assessment of image quality in 3D is achieved based on a coherence value of the image data generalized in an elevation dimension (rather than an azimuth dimension used for 2D ultrasound data). Thus, image quality values are determined for different parts of the 3D region based on the coherence factor of the associated ultrasound image data. From the image quality values, a volume of the 3D region that is adequately scanned may be determined. In this way, feedback regarding the acquisition quality of the 3D ultrasound scan may be provided.

For a 2D scan, a set of lines may be provided to delimit the part of the image that has a good image quality score. Part of the image may be shown as having a bad/insufficient image quality score, due to a poor acoustic window. The position of the lines may be updated as the transducer (i.e. the probe) of the ultrasound scanner is moved, thus providing real-time feedback indicating the part of the subject that is adequately imaged.

However, the benefit of providing the lines overlaid on a 2D ultrasound image is limited. This is because a sonographer can easily see from the image itself what part of the image is of adequate quality, and what part is not.

Conversely, for a 3D ultrasound scan, assessing and visualizing the quality of the image at every point in the scanned volume is no longer straightforward. Simply rendering the whole volume and providing the rendered 3D image will not provide the sonographer with a straightforward/easy way to determine the part that is adequately scanned or not. Not only is it difficult for the sonographer to determine, at a glance, which parts of the volume are adequately scanned, it is also problematic to actually display a 3D volume in a meaningful manner.

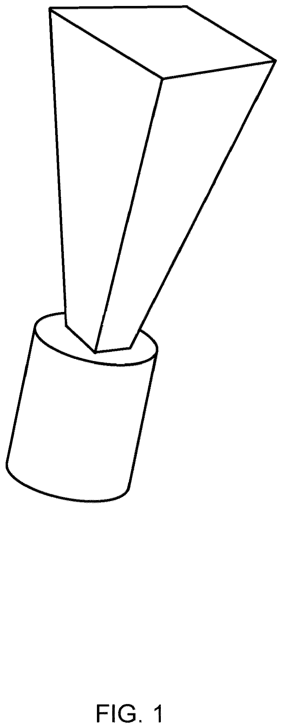

As illustrated in Fig. 1, and according to an aspect of the invention, it is proposed to generate a signal containing information for displaying a set of lines in 3D to the user/sonographer that show the extent of the acceptably scanned area (i.e. according to image quality values). Again, the position of the lines may update in real-time as the probe/transducer is moved, thus showing the user what part of the volume is inadequately or adequately scanned (such as due to blocking by a rib or lung when attempting to scan the heart, or by irregular positioning of the probe/transducer).

Furthermore, according to other aspects of the invention, the lines may be overlaid over a 3D representation of the volume of interest (e.g. the heart), to show what parts of the scanned area are blocked/inadequately scanned with respect to the volume of interest (e.g. the heart). As a result, it may be immediately apparent to the user which parts of the subject are being adequately scanned. Moreover, if it is not possible to capture/image/scan the whole region of interest in a single acoustic window, the scanning session may be performed in 2 iterations, with the lines guiding the position of the probe for each iteration.

In yet another embodiment of the aspect, a 3D live navigation schematic could be shown to the user during the acquisition. For example, the navigation schematic may present a representation of the subject's chest/ribcage in positional relation to the transducer, showing an estimate of the entry window and a graphical depiction of the 3D volume acquired during the scanning session. Also, various parts of the 3D volume could be represented by a number of 2D slices that are assessed to have a poor image quality.

Moving on, it is also challenging to determine/calculate an image quality value for each part of the 3D region scanned by the ultrasound scanner.

One method to assess image quality is through the coherence factor. The coherence factor is a measure of coherence of the ultrasound data, based on dividing the coherent intensity (coherent sum of the rf data on each channel of the ultrasound scanner), by the incoherent intensity (the sum of intensity of each channel). This is a well-known factor in ultrasound imaging, with various modifications of it well known to the skilled person.

However, the coherence factor is typically known for assessing the image quality of a 2D ultrasound scan. A 2D scan and corresponding coherence factor is based on focused transmission. Conversely, in 3D, unfocused beams in azimuth are typically utilized. Accordingly, calculating the coherence factor is more problematic. One method to calculate the coherence factor for 3D is to compute the coherence factor in the elevation dimension, where the beam is focused. This would assess the coherent sum across elevation, normalized by the sum of intensity in elevation.

Another method to overcome this problem is to retrospectively refocus the transmit beam first, and once the transmit beam is refocused, the coherence factor can be computed in azimuth, or in both azimuth and elevation.

To summarize, the coherence factor for 3D ultrasound data may be computer either by calculating the coherence factor in the elevation dimension, or by first refocusing the transmit beam before calculating the coherence factor. In essence the coherence factor provides an indication of the image quality in a part of the 3D volume (e.g. at each voxel). Parts of the 3D volume that are blocked are associated with a low coherence factor.

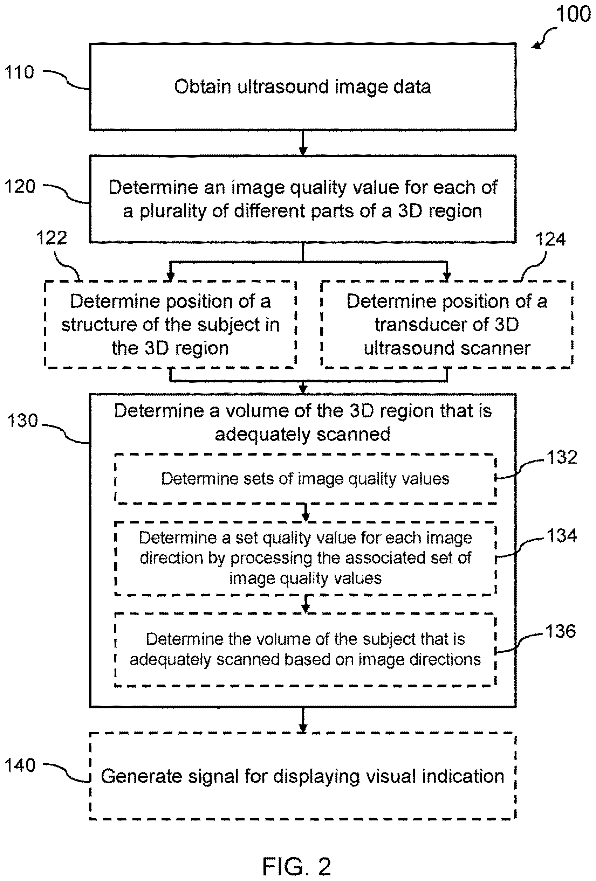

Turning to Fig.2, there is presented a flow diagram of a method for assessing acquisition quality of a scan of a subject by a 3D ultrasound scanner according to embodiments of the invention. That is, the method is suitable for aiding in the assessment/analysis of the quality of the ultrasound image data acquired during a scan of the subject. Such a method may be useful for users/sonographers to gain a better understanding of the quality of the scan that they are performing (i.e. whether or not the scan is being performed in such a way that an image of an area of interest of sufficient quality is acquired).

Firstly, in step 110, ultrasound image data associated with a 3D region of a subject is obtained. That is, ultrasound image data produced during a 3D ultrasound scan of the subject is obtained. The 3D region of the subject is a volume/region/area that is scanned by the 3D ultrasound scanner. For example, the 3D region may correspond to the heart of the subject, but may further comprise surrounding tissue/objects.

The ultrasound image data may be acquired from a 3D ultrasound scanning session in real-time. Accordingly, the method for assessing acquisition quality of the scan may be performed during the scanning session, and thus may provide feedback in real-time as described below. Of course, embodiments are not restricted to this, and the data may be performed after the fact, for post-hoc analysis and feedback.

In step 120, an image quality value is determined for each of a plurality of different parts of the 3D region. Each image quality value indicates a quality of the ultrasound image data associated with the part of the 3D region. Further, each image quality value is based on a coherence factor of the associated ultrasound image data generalized to an elevation dimension.

In other words, the 3D region may be considered to comprise a plurality of different parts/regions/areas. For each of these parts, an image quality value reflective of the quality of the image (i.e. the usability of the ultrasound image resultant from the ultrasound data corresponding to the area) is calculated.

Each image quality value is determined based on a calculated coherence value. The use of the coherence value is well-known in 2D as a metric to assess the quality of the ultrasound image data. Generally, it is the coherence intensity divided by the incoherent intensity. However, in the case of 2D the scan is based on focused transmission. This is not the case in 3D, with diverging beams being utilized.

Therefore, for the coherence factor to be meaningful, it must be generalized to the elevation dimension. In other words, the coherence factor in the case of the present invention may be the coherent intensity in elevation divided by the incoherent intensity in elevation. Alternatively, the transmit beam of the ultrasound scanner may be retrospectively refocused (thus generalizing to the elevation dimension) and the coherence factor calculated in the regular way.

As a result, a plurality of image quality values for different parts of the 3D volume are obtained. The different parts of the 3D volume, at it's most granular, may be voxels of the 3D region of the subject. This should not be considered restrictive, and sets of voxels may be represented by a single image quality value.

In step 130, a volume of the 3D region that is adequately scanned is determined. Parts of the 3D region that may be imaged/rendered in such a way so as to be useful for a user (e.g. for diagnosis/analysis) are identified in order to determine a volume of the region that has been adequately scanned. For example, such a volume may exclude any parts of the 3D region that are excessively noisy, distorted, blurry, or have low resolution, contrast, sharpness, or have a large number of artifacts. That is, if parts of the resultant imaged volume are excessively noisy, etc. then it may not be possible for a user to properly understand/interpret the image (i.e. the image may not be reflective of the condition of the subject in that volume).

The determination is based on identifying parts of the 3D region associated with image quality values meeting a predetermined requirement. The predetermined requirement may be based on at least one of a contrast value, a resolution value, a distortion value, a noise value, a sharpness value, and an artifact density value. Accordingly, the coherence factor may be adapted/interpreted in the previous step to reflect the extent of the above values, and compared to the predetermined requirement to inform a decision as to whether the part of the 3D region is of satisfactory quality.

In some embodiments, identifying parts of the 3D region comprises assessing each of the image quality values based on the predetermined requirement. That is, each image quality value is individually assessed against the predetermined requirement in order to identify parts of the 3D region that are adequately scanned. More specifically, it may be assessed whether the image quality value meets and/or exceeds a quality value defined by the predetermined requirement.

In an alternative embodiment, determining the volume that is adequately scanned comprises the following (optional) sub-steps.

Firstly, in sub-step 132, sets of image quality values are determined, each set of image quality values associated with parts of the 3D region in a different image direction. An image direction is a direction extending linearly from the transducer/probe head of the ultrasound scanner. In other words, an image direction is a direction of travel of an acoustic wave of the ultrasound device.

In sub-step 134, the associated set of image quality values are processed for each image direction. As a result, set quality values are determined indicating a quality of the ultrasound image data in each image direction. In its simplest form, a set quality value may simply be an average of the image quality values of the parts of the 3D region in an image direction. However, different image quality values may be provided with different weightings, or another statistical method for determining the set quality value may be used.

In sub-step 136, the volume of the subject that is adequately scanned is determined based on identifying image directions associated with set quality values meeting the predetermined requirement. Similarly to the above, set quality values are compared to the predetermined requirement to determine whether the parts of the 3D region have been adequately scanned in given directions. Overall, this may provide a set of image directions that are adequately scanned, and those that aren't. This may be relatively straightforward to display to a user, and for a user to understand.

In (optional) step 140, a signal comprising information for displaying a visual indication describing the volume of the 3D region is generated. The signal may be suitable for controlling a display to present a visual indication describing the adequately scanned volume of the 3D region. The visual indication may be any visual means by which a user can ascertain the volume that has been/is being adequately scanned.

In specific embodiments, the visual indication may comprise a set of lines representing boundaries of the volume. This may be like that seen in Fig. 1, with a simple set of lines delimiting the region that is adequately scanned, and reduced or increased as specific parts of the volume are imaged adequately or inadequately.

Additionally, the visual indication may comprise an image representing at least a part of the 3D region of the subject overlaid by the set of lines. The image may represent the anatomy of the subject being imaged. For example, the image may be of the heart, such that the lines clearly indicate which parts of the heart have been (in)adequately imaged.

In (optional) step 122, the ultrasound image data is processed to determine a position of a structure of the subject in the 3D region obstructing ultrasound imaging of the subject. The processing may be performed using any known method, such as by processing B-mode components of the ultrasound image data based on an image analysis algorithm, and/or processing channel components of the ultrasound image data based on a signal processing algorithm.

Accordingly, determining the volume of the 3D region is further based on the determined position of the structure. That is, the position of the structure may dictate which parts of the 3D region have been adequately scanned.

For example, the structure may comprise at least one rib of the subject, or may comprise a lung when a region of interest is the heart. Other structures would be readily appreciated by the skilled person. If a certain structure is in between the transducer head/probe and the part of the 3D region, then it is unlikely that the part of the 3D region has been adequately scanned.

In (optional) step 124, the ultrasound image data is processed to determine a position of a transducer of a 3D ultrasound scanner used to acquire the ultrasound image data relative to the 3D region of the subject. Accordingly, the volume of the 3D region is determined based also on the determined position of the transducer.

The position may include the position of the transducer/probe hear relative to a surface of the subject, as well as an orientation of the transducer. For example, if the transducer is separated from the surface of the subject, an image quality may be reduced.

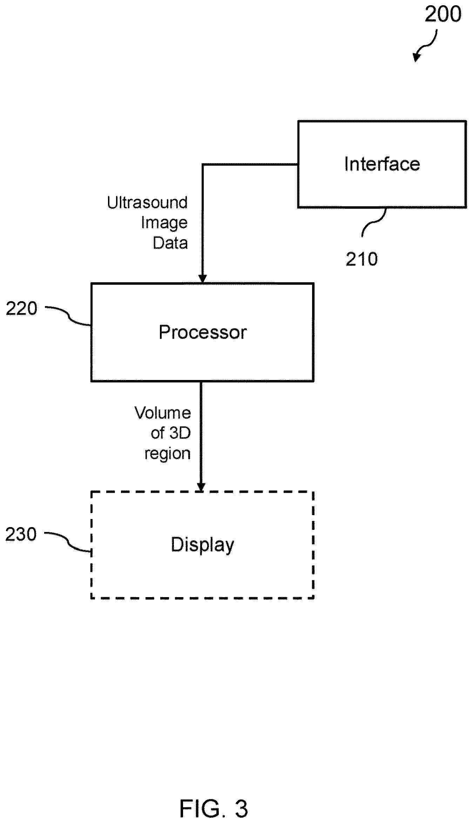

Fig.3 presents a simplified block diagram of a system 200 for assessing acquisition quality of a scan of a subject by a 3D ultrasound scanner. Specifically, the system 200 comprises an interface 210 and a processor 220, and may further (optionally) comprise a display 230.

The interface 210 is configured to obtain ultrasound image data associated with a 3D region of a subject. This may be obtained directly from the 3D ultrasound scanner, or may be obtained indirectly from a database or other memory storage. The interface 210 passes said information to the processor 220.

The processor 220 is configured to determine an image quality value for each of a plurality of different parts of the 3D region. Similarly to the above, the image quality value indicates a quality of the ultrasound image data associated with the part of the 3D region, and each image quality value is based on a coherence factor of the associated ultrasound image data generalized to an elevation dimension.

Furthermore, the processor 220 is configured to determine a volume of the 3D region that is adequately scanned based on identifying parts of the 3D region associated with image quality values meeting a predetermined requirement, similarly to the above described method.

The processor 220 may also be configured to perform any of the steps 120-140 described in relation to Fig.2. Specifically, the processor 220 may also be configured to generate a signal comprising information for displaying a visual indication describing the volume of the 3D region. The signal may be suitable for controlling a display 230 to present a visual indication describing the adequately scanned volume of the 3D region.

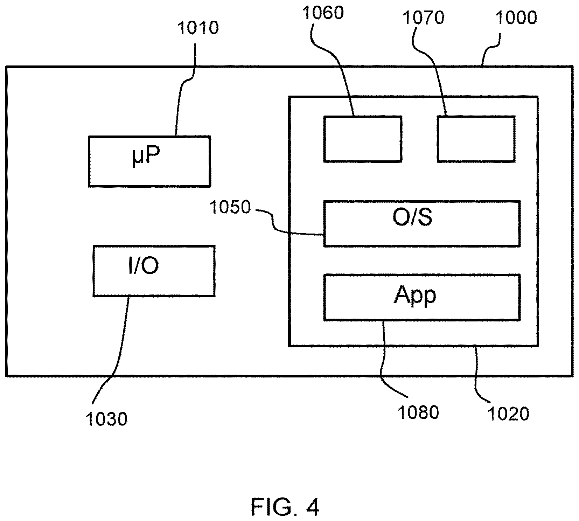

Thus, the processor may then transmit the signal to the display 230. The display 230 may be configured to present an indication of the adequately scanned volume to a user/sonographer.
Moving on, Fig.4 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method described in relation to Fig.2, and the system described in relation to Fig.3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram Fig.3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for assessing acquisition quality of a scan of a subject by a 3D ultrasound scanner, comprising:
obtaining (110) ultrasound image data associated with a 3D region of a subject;
determining (120), for each of a plurality of different parts of the 3D region, an image quality value indicating a quality of the ultrasound image data associated with the part of the 3D region, each image quality value based on a coherence factor of the associated ultrasound image data generalized to an elevation dimension; and
determining (130) a volume of the 3D region that is adequately scanned based on identifying parts of the 3D region associated with image quality values meeting a predetermined requirement.

2. The method of claim 1, further comprising:
generating (140) a signal comprising information for displaying a visual indication describing the volume of the 3D region.

3. The method of claim 2, wherein the visual indication comprises a set of lines representing boundaries of the volume.

4. The method of claim 3, wherein the visual indication comprises an image representing at least a part of the 3D region of the subject overlaid by the set of lines.

5. The method of any of claims 1-4, wherein the predetermined requirement is based on at least one of a contrast value, a resolution value, a distortion value, a noise value, a sharpness value, and an artifact density value.

6. The method of any of claims 1-5, wherein identifying parts of the 3D region comprises assessing each of the image quality values based on the predetermined requirement.

7. The method of any of claims 1-5, wherein identifying parts of the 3D region comprises:
determining (132) sets of image quality values, each set of image quality values associated with parts of the 3D region in a different image direction;
for each image direction, processing (134) the associated set of image quality values to determine a set quality value indicating a quality of the ultrasound image data in the image direction; and
determining (136) the volume of the subject that is adequately scanned based on identifying image directions associated with set quality values meeting the predetermined requirement.

8. The method of any of claims 1-7, further comprising processing the ultrasound image data to determine (122) a position of a structure of the subject in the 3D region obstructing ultrasound imaging of the subject, wherein determining (130) the volume of the 3D region is further based on the determined position of the structure.

9. The method of claim 8, wherein determining (122) the position of the structure comprises processing B-mode components of the ultrasound image data based on an image analysis algorithm, or processing channel components of the ultrasound image data based on a signal processing algorithm.

10. The method of claims 8 or 9, wherein the structure comprises at least one rib of the subject.

11. The method of any of claims 1-10, further comprising processing the ultrasound image data to determine (124) a position of a transducer of a 3D ultrasound scanner used to acquire the ultrasound image data relative to the 3D region of the subject; wherein determining (130) the volume of the 3D region is further based on the determined position of the transducer.

12. The method of any of claims 1-11, wherein each of the plurality of different parts of the 3D region of the subject are voxels of the 3D region of the subject.

13. The method of any of claims 1-12, wherein obtaining (110) the ultrasound image data comprises acquiring the ultrasound image data from a 3D ultrasound scanning session in real-time, and wherein determining (130) the volume is performed during the scanning session.

14. A computer program comprising computer program code means adapted, when said computer program is run on a computer, to implement the method of any of claims 1-13.

15. A system for assessing acquisition quality of a scan of a subject by a 3D ultrasound scanner, comprising:
an interface (210) configured to obtain ultrasound image data associated with a 3D region of a subject; and
a processor (220) configured to:
determine, for each of a plurality of different parts of the 3D region, an image quality value indicating a quality of the ultrasound image data associated with the part of the 3D region, each image quality value based on a coherence factor of the associated ultrasound image data generalized to an elevation dimension; and
determine a volume of the 3D region that is adequately scanned based on identifying parts of the 3D region associated with image quality values meeting a predetermined requirement.
